# EUROPEAN PATENT APPLICATION

(11) **EP 4 559 473 A1**
(43) Date of publication of application: **28.05.2025**
(21) Application number: 23768130.9
(22) Date of filing: 04.07.2023
(51) Int. Cl.: A61K 38/17, A61P 3/06, A61P 3/08, A61P 9/10

(54) **PEPTIDE FOR THE TREATMENT OF DISEASES ASSOCIATED WITH APOLIPOPROTEIN AI OR TRANSTHYRETIN INVOLVEMENT**

(30) Priority: 22.07.2022 CU 20220040
(71) Applicant: Centro de Ingeniería Genética y Biotecnología, La Habana 11300 (CU)
(72) Inventor: DOMÍNGUEZ HORTA, Maria del Carmen, La Habana 11300 (CU); RAMOS GÓMEZ, Yassel, La Habana 11300 (CU); HERRERA WONG, Mónica, La Habana 10900 (CU); MARTÍNEZ DONATO, Gillian, La Habana 11300 (CU); CHINEA SANTIAGO, Glay, La Habana 11300 (CU); GUILLÉN NIETO, Gerardo, Enrique, La Habana 11300 (CU); VENEGAS RODRÍGUEZ, Rafael, La Habana 11700 (CU); UBIETA GÓMEZ, Raimundo, La Habana 11300 (CU); GONÁLEZ BLANCO, Sonia, La Habana 11300 (CU); PAEZ MEIRELES, Rolando, La Habana 17100 (CU); SERRANO DÍAZ, Anabel, La Habana 11000 (CU)
(74) Representative: Capitán García, Maria Nuria
(86) International application number: PCT/CU2023/050002
(87) International publication number: WO 2024/017423

(57) **Abstract**

Peptide identified as SEQ ID NO: 1, for use in the manufacture of a medicine for treatment of a disease involving an affection of apolipoprotein A-I (Apo-AI) or transthyretin (TTR). In particular, the peptide may be useful for treatment of a disease characterized by an impairment in lipid homeostasis or in the stability of TTR. Method of treatment of a disease involving an affection in Apo-AI or TTR, which is characterized by administration of a therapeutically effective amount of a pharmaceutical composition comprising the peptide identified as SEQ ID NO: 1.

## Description

### Technique field

The present invention narrates to the branch of medicine, in particular with a modified APL type peptide (Altered Peptide Ligand, abbreviated APL), derived from HSP60 and its use to ensure lipid homeostasis, prevent cellular oxidative stress, and avoid amyloidosis caused by inappropriate modifications of apolipoprotein A-I (Apo-AI) and transthyretin (TTR). The peptide is also useful for the treatment of patients with impaired lipid transport and processing.

### The prior state of the art

Lipids are essential for the functioning of the human organism, and these molecules are crucial in obtaining and storing energy. In addition, lipids are fundamental components of lipid membranes, various hormones, and bile salts. Imbalances in lipid concentration, either due to excess or deficiency, are directly related to the development of cardiovascular diseases, such as coronary disease, cerebrovascular disease, or dyslipidemia (Krahmer, N. et al. (2013) EMBO Mol. Med. 5, 973-983).

Lipids are hydrophobic biomolecules, and most of them are insoluble in blood, so their transport is driven by lipoproteins. Lipoproteins are classified based on their size and density. Lipoproteins are hydrophobic spherical structures, which have proteins on their surface (apoproteins or apolipoproteins), capable of binding the enzymes in charge of lipid processing. (Rosenson, R.S. et al. (2016). Nat. Rev. Cardiol. 13, 48-60).

High-density lipoproteins (HDL) transport excess cholesterol from extrahepatic tissues to the liver, where it is metabolized. HDL are related to a decrease in cardiovascular risk, and low-density lipoproteins (LDL) and very low-density lipoproteins (VLDL) increase it. There is an inverse correlation between the concentration of cholesterol associated with HDL and the risk of atherosclerosis. (Lee, M., P. T. et al. (2003). J. Lipid Res. 44: 539-546).

HDL are the highest-density lipoproteins because they have higher concentration of proteins than lipids. These lipoproteins are composed of cholesterol, triglycerides, and numerous apolipoproteins: Apo-AI, Apo-AII, Apo-AIV, Apo-AV, Apo-C1, Apo-CII, Apo-CIII, and Apo-E. Apo-AI is the main protein component of HDL and is the primary apolipoprotein responsible for maintaining the structure of HDL (Jonas, A., K. E. Kezdy, y J. H. Wald. (1989). J. Biol. Chem. 264: 4818-4824).

Apo-AI activates the enzyme lecithin-cholesterol-acyl-transferase, which catalyzes the transformation of cholesterol to its ester form, thereby increasing the ability of HDL to transport lipids to the liver (Gordon, T. et al. (1977). Am. J. Med. 62, 707-714). The blood concentration of Apo-AI increases during certain physiological conditions such as pregnancy, liver disease, and estrogen use, and decreases during sepsis and atherosclerosis. High concentrations of Apo-AI and low concentrations of Apo-B (the main protein component of low-density lipoproteins) are significantly correlated with a reduced risk of cardiovascular disease. HDL have anti-atherogenic and antiinflammatory properties, since they transport cholesterol accumulated in atheromatous plaques to the liver, thus contributing to the reduction of said plaques (T.M. Forte, et al. (2002) J Lipid Res 43: 477-485).

The mechanisms of formation of atherosclerotic plaques are not fully understood, but LDL and HDL lipoproteins play a fundamental role in these mechanisms. Until now, no effective treatment has been found to prevent the formation of atheromas and prevent the consequent damage to the human body. Therefore, obtaining treatments that guarantee lipid homeostasis and proper processing of lipoproteins is crucial.

On the other hand, it has been described that TTR forms part of HDL, through binding to Apo-AI (Liz, M. A., C. M. Gomes, M. J. Saraiva, and M. M. Sousa. (2007) J Lipid Res 48: 2385-2395). TTR is a tetrameric protein present in plasma, synthesized mainly in the liver, and is responsible for the transport of thyroxine and retinol-binding protein. This protein can be broken down into monomers and dimers, which can be attached to fibers and deposited in tissues.

Age and mutations (more than a hundred have been identified) can increase the predisposition to aggregation. Two forms of TTR amyloidosis have been described: the natural form (wt ATTR, wild type transthyretin cardiac amyloidosis, known as senile systemic amyloidosis) and the hereditary form (h-ATTR, hereditary transthyretin cardiac amyloidosis). In the natural form, the synthesis of TTR is normal, while in the hereditary the presence of a mutation affects the conformation of TTR since its synthesis in hepatocytes (Kittleson MM, Maurer MS, Ambardekar AA, et al. al (2020) Circulation 142:e7-22).

Among the diseases caused by TTR amyloidosis is amyloid cardiomyopathy, which refers to heart disease resulting from the extracellular deposition of abnormal insoluble fibrils. The prevalence of this disease is estimated between 13%, in hospitalized older adults due to preserved ejection fraction, and 16% of severe aortic stenosis that requires intervention (Coelho T, Maurer MS, Suhr OB. (2013) Curr Med Res Opin; 29:63-76).

In addition, post-translational modifications of the TTR that affect its tetrameric conformation, and the presence of its oligomers, are strongly related to Parkinson's disease (Ando Y, Nakamura M and Araki S. (2005) Arch Neurol 62: 1057-1062), and with pathologies associated with oxidative stress (Sharma M, Khan S, Rahman S and Singh LR (2019) Front. Physiol. 10:5. doi: 10.3389/fphys.2019.00005).

It has been described that a fraction of plasmatic TTR circulates in HDL through binding to Apo-AI. TTR is capable of cleaving the carboxyl terminus of lipid-free Apo-AI. Cleavage of Apo-AI by TTR induces the formation of Apo-AI amyloid fibrils. Furthermore, it causes the modified HDL particles to have a reduced ability to promote proper cholesterol turnover. In addition, Apo-AI cleaved by TTR has a high preference for forming aggregated particles, which affects HDL turnover, and favors the development of atherosclerosis, by reducing cholesterol metabolism and increasing the amyloidogenic potential of Apo-AI (Liz, M.A., C.M. Gomes, M.J. Saraiva, and M.M. Sousa. (2007) J. Lipid Res. 48: 2385-2395).

The search for treatments that stabilize HDL is crucial to ensure adequate lipid exchange, especially cholesterol, and prevent the formation or reduction of atheromatous plaques that promote atherosclerosis. At the same time, treatments that stabilize the TTR conformation, preventing its modifications and aggregation, would be effective in preventing TTR-mediated amyloidosis and Parkinson's disease.

### Explanation of the invention

The present invention resolves the problem mentioned above, by providing a peptide whose sequence is identified as SEQ ID NO: 1, for its use in the manufacture of a medicine for the treatment of a disease that affects Apo-AI or in the TRT. The modified peptide identified as SEQ ID NO: 1 is capable of stabilizing the supramolecular structure of HDL, through binding to Apo-AI. In this way, it facilitates an adequate metabolism of lipids and the reduction of atherosclerotic plaques, associated with atherosclerosis. As shown in the invention, surprisingly, this peptide only interacts with Apo-AI and TTR in human plasma. This interaction allows for the stability of both proteins.

In the context of the invention, "a disease that affects Apo-AI or TTR" is defined as a disease in which there is an increase or decrease in the plasma concentration of Apo-AI or TTR, or in which modifications occur in the interactions of these proteins in HDL particles, or those diseases where the structures of Apo-AI and TTR are affected, and therefore their biological functions.

Previously, it was described that the peptide identified as SEQ ID NO: 1 induces regulatory mechanisms of the immune response in different experimental systems. Said peptide increased the frequency of regulatory T cells (Tregs) in ex vivo assays with peripheral blood mononuclear cells from patients with rheumatoid arthritis (RA), but not in healthy donors; these cells have suppressive activity (Barberá A, Lorenzo N, van Kooten P, et al. (2016) Cell Stress and Chaperones.; 21:735-744). Likewise, said peptide induced a significant increase in the Treg cell population in BALB/c mice. Furthermore, this peptide efficiently inhibited RA in two animal models (Lorenzo N, Altruda F, Silengo L and Dominguez MC. (2017) Clin Exp Med; 17:209-216). Treatment with the peptide identified as SEQ ID NO: 1 in patients with RA, proved to be safe and had a good therapeutic effect (Dinorah Prada, Jorge Gómez, Norailys Lorenzo, Oreste Corrales, et al. (2018) Journal of Clinical Trials; 8:2167-0870, Cabrales-Rico, A., Ramos, Y., Besada, V., Del Carmen, D. M., Lorenzo, N. et al.(2017) J Pharm.Biomed.Anal.143:130-140).

These results were disclosed in international patent applications No. PCT/CU2005/000008 and PCT/CU2009/000009. These claim the peptide identified as SEQ ID NO: 1 and its use for the treatment of RA and Crohn's disease, ulcerative colitis, and type I diabetes mellitus, respectively.

Subsequently, the international patent application No. PCT/CU2018/050007 claimed a very stable pharmaceutical composition containing the peptide of SEQ ID NO: 1, for the treatment of diseases characterized by increased citrullination and the number of neutrophils. In particular, said invention relates to the treatment of RA, ankylosing spondylitis, juvenile idiopathic arthritis, liver and lung fibrosis, and Alzheimer's disease. On the other hand, the peptide identified as SEQ ID NO: 1 has been used in the treatment of patients with hyperinflammation, as is the case of patients with COVID-19; and it was revealed to reduce inflammation in such patients (Hernéndez-Cedeño M et al. (2021). Cell Stress and Chaperones 26: 515-525; Domínguez Horta MC, et al. (2022). Annals of the Academy of Sciences of Cuba; 12(1): e1072. http://www.revistaccuba.cu/index.php/revacc/article/view/1072). This fact was first revealed in the international patent application No. PCT/CU2021/050001, which claims the use of said peptide for the treatment of hyperinflammation.

The present invention demonstrates, for the first time, the ability of the peptide of SEQ ID NO: 1 to strongly bind to the APO-AI and TTR proteins, which contributes to guaranteeing stable lipid homeostasis and reducing amyloidosis processes mediated by said proteins. In addition, this invention demonstrates the benefit received by patients treated with the peptide identified as SEQ ID NO: 1, who had reached a state of seriousness due to diseases related to lipid metabolism.

In an embodiment of the invention, the disease progress showing variations in Apo-AI or in TTR is characterized by a compromise in lipid homeostasis or in the stability of TTR. In a particular embodiment, said disease is selected from the group consisting of atherosclerosis, ischemic heart disease, type II diabetes, secondary dyslipidemia, and metabolic syndrome. In another particular embodiment, said disease is TTR amyloidosis or Parkinson's disease.

The peptide identified as SEQ ID NO: 1 reduces oxidative stress and significantly improves the condition of patients who are clinically severe due to cardiovascular diseases, such as ischemic heart disease, and hypertension. Treatment with this peptide in patients with ischemic heart disease and type II diabetes was very safe since it did not cause adverse effects.

The same peptide is known to be useful for the treatment of patients with type I diabetes. However, such use is based on the regulation of the uncontrolled immune response that persists in patients with type I diabetes. Type II diabetes is not considered an autoimmune disease. Surprisingly, treatment of patients with decompensated type II diabetes with said peptide allowed very rapid recovery, compared to patients who received standard treatment alone. In the present invention, it is unexpectedly shown that a very beneficial effect is produced in patients with type II diabetes, where treatment with the peptide identified as SEQ ID NO: 1 contributes to lipid homeostasis and its effect on intermediary metabolism.

The invention also provides a method of treating a disease that occurs with an affectation in Apo-AI or in TTR in an individual in need thereof. Said method of treatment is characterized in that a therapeutically effective amount of a pharmaceutical composition comprising the peptide identified as SEQ ID NO: 1 is administered. In one embodiment of the invention, the disease is selected from the group consisting of atherosclerosis, ischemic heart disease, type diabetes II, secondary dyslipidemia and metabolic syndrome. In another embodiment, the disease is TTR amyloidosis or Parkinson's disease.

In an embodiment of the invention, the pharmaceutical composition is administered by systemic route. In a preferred embodiment, the composition is administered by the subcutaneous or intravenous route. In an embodiment of the invention, said pharmaceutical composition is administered simultaneously with the standard therapy, during the same treatment.

### Brief description of the figures:

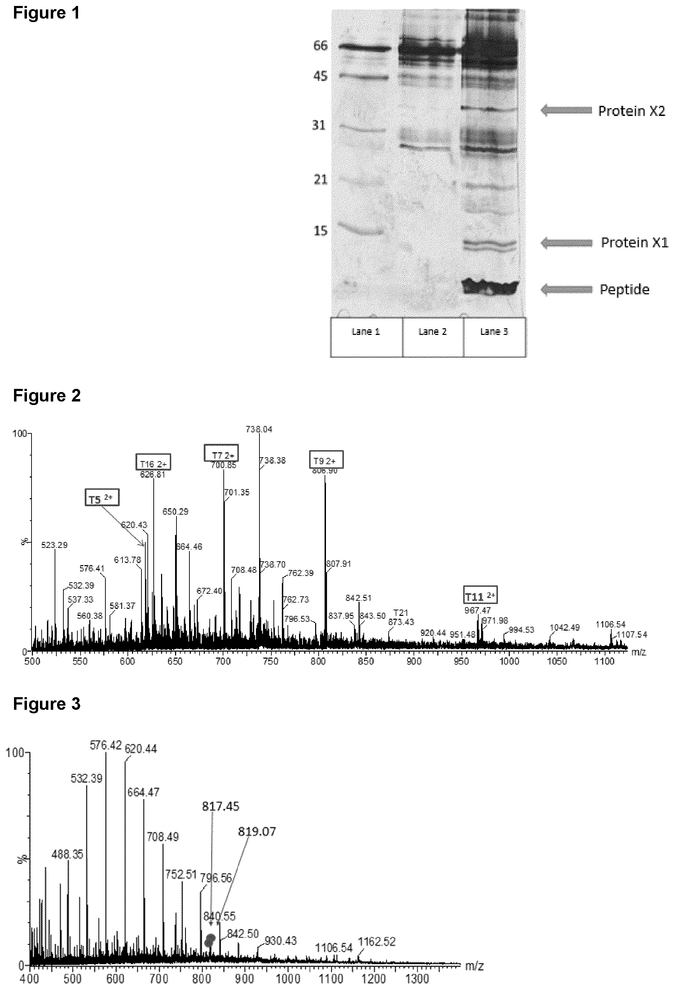

**Figure 1****.** SDS-PAGE electrophoresis showing proteins in human plasma that specifically bind to the peptide identified as SEQ ID NO: 1. Lane 1: molecular weight standard. Lane 2: matrix-binding proteins-no peptide (control). Lane 3: proteins that specifically bind to the peptide identified as SEQ ID NO: 1 bound to the matrix.
**Figure 2****.** Mass spectrum (MS) of the band identified as Apo-AI on SDS-PAGE. The boxes indicate the peptides that were identified with the MASCOT program in the UniProtKB database as constituents of Apo-AI.
**Figure 3****.** MS of the band identified as TTR in the SDS-PAGE. The dots indicate the peptides that were identified with the MASCOT program in the UniProtKB database as constituents of the TTR.

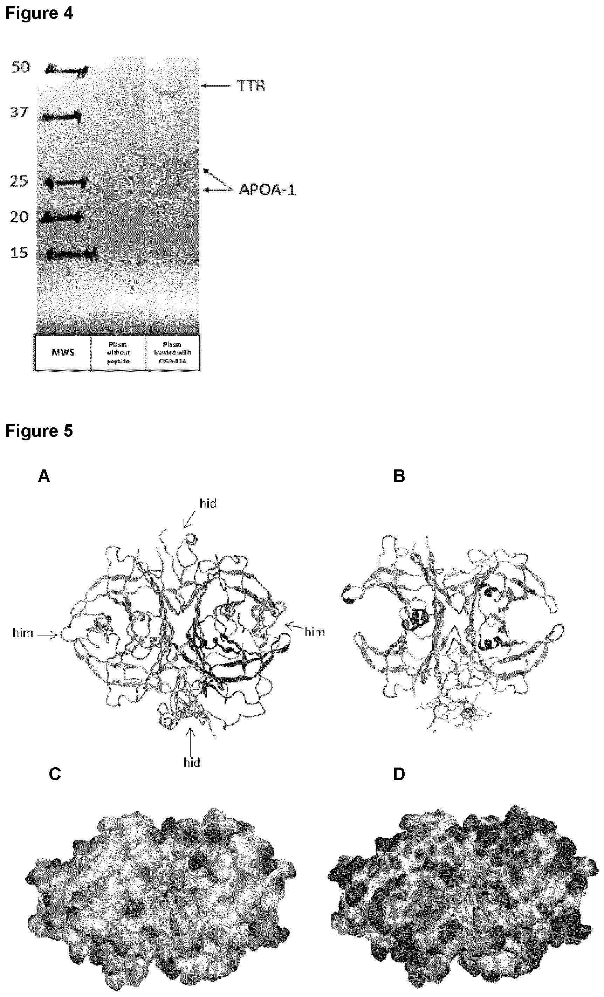

**Figure 4****.** Western Blot showing the proteins that specifically bind to the peptide identified as SEQ ID NO: 1 in human plasma. Lane 1: molecular weight standard. Lane 2: plasma that was not incubated with the peptide. Lane 3: plasma that was incubated with the peptide.

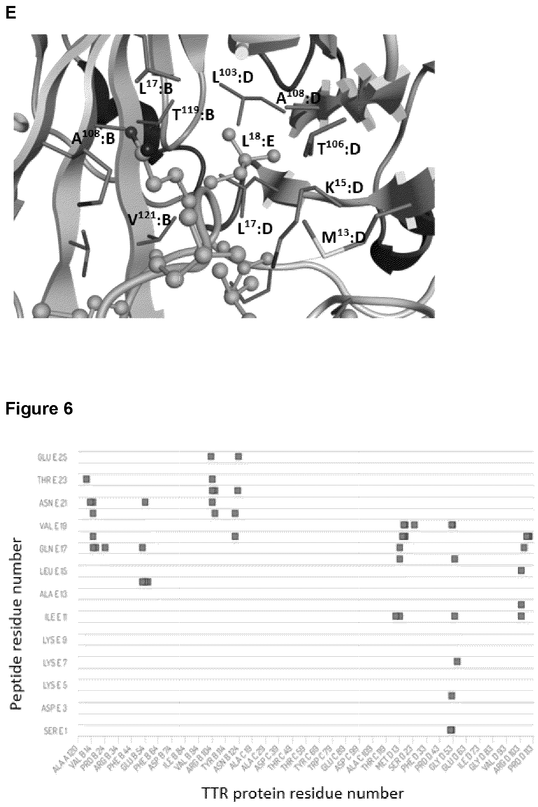

**Figure 5****. A,** Superposition of the structure of the ten highest scoring peptide-protein complex models predicted by the CABS-dock method. **B,** a model of the complex structure of the peptide identified as SEQ ID NO: 1, bound to the TTR protein to the "idc" site. **C,** representation of the peptide bound to TTR protein, showing the surface of the protein according to the hydropathic character. **D,** figure similar to C, but without representing the electrostatic character of the surface of the protein. **E,** view of interactions observed in the environment of the residue Leu18 of the peptide.
**Figure 6****.** Contact map among residues of the peptide identified as SEQ ID NO: 1 (vertical axis) and TTR protein (horizontal axis), according to the 3D structure prediction of the complex. The squares mean that the corresponding residues contain atoms located at a distance of 4.5 Å or less. The type of residue is shown in three-letter code, next to the chain identifier (chains B and D correspond to the TTR protein and the E chain to the peptide identified as SEQ ID NO: 1) and to the residue number of the amino acid sequence.

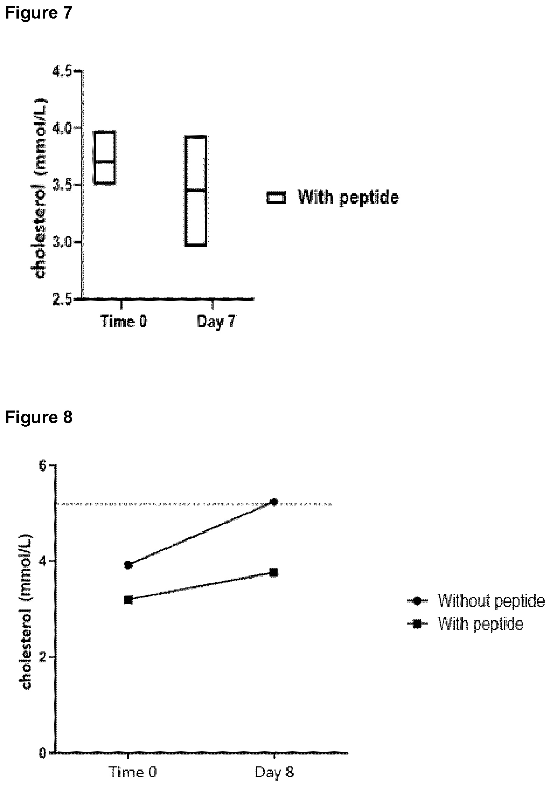

**Figure 7****.** Cholesterol levels in patients with ischemic heart disease and high blood pressure treated with the peptide identified as SEQ ID NO: 1. Time 0: cholesterol levels before starting the peptide treatment; day 7: cholesterol levels seven days after starting peptide treatment.
**Figure 8****.** Cholesterol levels in diabetic type II patients, five of whom received peptide treatment identified as SEQ ID NO: 1, compared to five patients not treated with the peptide. The discontinuous line marks the normal limit of blood cholesterol levels (5.20 mmol/L). Time 0: cholesterol levels before starting peptide treatment, day 8: cholesterol levels eight days after starting peptide treatment.

### Detailed presentation of modes of embodiment /Examples of embodiment.

### Example 1. Identification of apolipoprotein A (Apo-AI) as a protein binding the peptide identified as SEQ ID NO: 1 in human plasma.

In order to investigate the ability of the peptide identified as SEQ ID NO: 1 to bind plasma proteins, an affinity chromatography was performed using a chromatographic resin to which the synthetic peptide identified as SEQ ID NO: 1 was attached. First, blood was collected from a healthy donor, female, 24 years old, without any pathological connection and with a normal lipid profile. Blood was diluted 1:2 with PBS 1X and added to 3 mL of Ficoll-Paque^{™}, and centrifuged for 30 min at 1200 rpm. The plasma was collected, and incubated with 100 µL of the chromatographic matrix. This matrix is a pearl-shaped resin, called ChemMatrix^{™} (Quebec, Canada), to which the peptide identified as SEQ ID NO: 1 was previously attached (this union is hereinafter referred to as matrix-peptide).

Prior to incubation, the matrix-peptide was treated with a 70% methanol solution and RPMI medium culture. The matrix-peptide incubation with plasma was performed for 3 hours. Subsequently, elution was carried out with a solution containing: glycerol 20%; TRIS-HCI 62.5 mM; bromophenol blue 2,5%; SDS 2% and β-mercaptoethanol 5%; and the elution fraction was collected. It was heated to 95 °C by 5 min, and analyzed by SDS-PAGE electrophoresis (gel at 15%). As a control of the experiment, the procedure described above was performed with the ChemMatrix^{™} without attaching the peptide identified as SEQ ID NO: 1. The gel was stained with a *Coomassie* blue solution, this staining is compatible for protein identification by mass spectrometry.

Figure 1 shows the result of the analysis by SDS-PAGE electrophoresis, of the fractions collected after elution. The gel was subjected to silver staining. As can be clearly seen, two plasma proteins were detected that bind to the peptide identified as SEQ ID NO: 1, which were named as protein X1 and X2.

For identification of the X1 protein observed in SDS-PAGE, the corresponding band was cut, and 1 mL was added from a solution containing ammonium bicarbonate (BCA) 250 mM, acetonitrile (ACN) 30%, dithiothreitol (DTT) 1% in water. The sample was shaken for 15 minutes. Subsequently, this solution was removed and 1 mL was added from a solution containing BCA 250 mM; ACN 30%; acrylamide 2,5% in water. The sample was shaken for 15 minutes. Then, this solution was removed, and 1 mL was added from a solution containing BCA 250 mM, ACN 30%, acrylamide 2,5% in water. The sample was shaken for 15 minutes. Immediately, this solution was removed, and 1 mL was added from a solution containing BCA 250 mM and ACN 30% in water. The sample was shaken for 15 minutes. Next, three washes with water were made for 10 min under agitation and the band was cut into 1 mm³ cubes. 400 mL of pure ACN was added, homogenized using a vortex, and the supernatant was removed. The sample was then dried in a Speed Vac for 5 min, and the gel was hydrated with a 50 mM BCA solution containing trypsin at a concentration of 12.5 ng/mL. Incubation with trypsin was performed for 16 h at 37 °C. The gel was then rehydrated with water for 30 min at 37 °C.

Consecutively, the sample was desalted in a ZipTip C18^{™} microcolumn (Millipore, USA) and eluted with 3,5 µL of ACN 60% in water containing 1% formic acid. The sample was applied, in a borosilicate capillary coated with a conductive material (Thermo Scientific, USA), and analyzed by nano ESI-MS. The ESI-MS spectra were obtained in an orthogonal hybrid configuration spectrometer QTOF-2 (Micromass, United Kingdom) with a Z-spray electrospray ionization source in positive mode (nanoESI+). The analyzer was calibrated with a mixture of sodium and caesium iodide (Sigma, USA) as a reference in a broad mass range (50-2000 Th). The ESI-MS spectra were obtained by applying a voltage of 1200 volts and 30 volts in the capillary and in the input cone of the mass spectrometer, respectively. MassLynx version 4.1 (Micromass, United Kingdom) was the software used for mass spectrum acquisition and processing. The ESI-MS/MS spectra were obtained by applying a collision energy in the range of 20-40 eV, to induce fragmentations containing sufficient information for the structural elucidation of the peptides analyzed. Argon was used as collision gas (Gases Industriales, Cuba).

The ESI-MS spectrum of tryptic digestion of the protein corresponding to the band identified as X1 showed multi-charged ions (2+) corresponding to five peptides (m/z 620.43; 626,81; 700,85; 806.90 and 967.47), which were selected to fragment and obtain the ESI-MS/MS spectra. These species correspond to the peptide fragments ³⁷DLATVYVDVLK⁴⁸, ⁵³DYVSQFEGSALGK⁶⁶, ⁷²LLDNWDSVTSTFSK⁸⁶, ⁸⁹EQLGPVTQEFWONLEK¹⁰⁴, and ¹²³VQPYLDDFQK¹³³, of the protein Apo-AI, identified by the MASCOT software searching the UniProtKB database (Figure 2).

### Example 2. Identification of transthyretin as a protein binding the peptide identified as SEQ ID NO: 1 in human plasma.

For identification of the protein X2 observed in the SDS-PAGE, we proceeded as in Example 1. The ESI-MS spectrum of the protein of the upper band showed the multi-charged ions (3+) corresponding to two peptides (*m*/*z* 819.07 and 817.45; respectively) that were selected for fragmentation using CID (Collision-Induced *Dissociation*)*.*

These species correspond to the peptide fragments ³⁶KTSESGELHGLTTEEEFVEGIYKV⁶⁹ and ⁶⁸KALGISPFHEHAEVVFTANDSGPRR⁹², respectively, of the TTR protein identified by the MASCOT program searching the UniProtKB database. The ESI-MS spectrum of the protein is depicted in Figure 3.

### Example 3. Immune recognition of the peptide identified as SEQ ID NO: 1 bound to apolipoprotein-Al or transthyretin.

To confirm that the peptide identified as SEQ ID NO: 1 binds to the Apo-AI and TTR proteins present in plasma, a *Western* blot type immunoidentification experiment was conducted with polyclonal antibodies against this peptide. The serum of a rabbit previously immunized with the peptide identified as SEQ ID NO: 1, conjugated to the protein KLH (antibody vs peptide), was used as the primary antibody. During the biological characterization of the polyclonal serum used, it was confirmed that it does not recognize Apo-AI or TTR. First, an SDS-PAGE electrophoresis (12,5% gel) was performed, where the plasma of a healthy donor was applied in two lanes and a molecular weight standard in another one. Subsequently, the transfer of the proteins present in the gel was carried out, to a nitrocellulose membrane and stained with a Red *Ponceau* solution, and the bands corresponding to the molecular weight standards were marked. The membrane was cut, according to the lanes where the plasma was applied. Membrane fragments were treated with a 1% bovine serum albumin solution containing 0,05% *Tween* 20; at 4 °C, overnight.

Next, one of the membrane fragments was incubated with a peptide solution identified as SEQ ID NO: 1, at a concentration of 200 µg/mL in PBS 1X plus *Tween* 20 (0,05%). This incubation was carried out for three hours at 37 °C, the remaining membrane fragments were treated equally, but without adding the peptide to the solution. Subsequently, membrane fragments were washed with a solution composed of PBS 1X plus *Tween* 20 (0,05%). The membrane fragments were then incubated with polyclonal serum vs. peptide as primary antibody. The polyclonal serum was diluted 1/2500 in the washing solution and incubation was performed for two hours at 37 °C. After that time, washes were repeated and the secondary antibody was added (Sigma, USA), which consists of a mixture of G-type immunoglobulins that recognize the constant region of rabbit antibodies, conjugated to horseradish peroxidase. Incubation with the secondary antibody was performed for one hour at 37 °C. Washing was then carried out, and development was performed with 0,01% hydrogen peroxide and 3.3 '-diaminobenzidine (1 mg/mL).

The results are shown in Figure 4. It is observed the immune recognition by the antibody vs peptide at the height of the bands corresponding to Apo-AI and TTR. This experiment confirms the results shown in examples 1 and 2, and therefore confirms that the peptide identified as SEQ ID NO: 1 interacts with these two proteins present in human plasma.

### Example 4. Modeling of the structure of the complex formed between the peptide identified as SEQ ID NO: 1 and the protein Transthyretin.

Monomeric TTR adopts a beta sandwich type folding consisting of two antiparallel beta sheets. The native TTR protein is characterized by adopting a quaternary tetrameric structure, which is established by the association of two dimers of the protein. The dimer of TTR is initially configured by the interaction of the strands of one end of the beta-sheet of the monomeric protein, resulting in the formation of two extended antiparallel beta-sheets. The convex surface of one of the sheets of each dimer interacts face to face with the analogous extended sheet of the other dimer, and the tetramer is obtained. In the space between these sheets, a cavity or canal is created that accommodates the natural ligand (T4), as well as other drug-like small molecules (Cotrina et al. (2021). European Journal of Medicinal Chemistry. Vol 226, p. 113847). The inter-dimers interaction is relatively unstable, and tetramer dissociation is related to a number of pathologies associated with the TTR protein, such as TTR amyloidosis (Ando Y, Nakamura M, and Araki S. (2005) Arch Neurol 62: 1057-1062). The binding site described in the space located between the dimers is the target of drugs studied and/or developed against several of these diseases.

The CABS-dock flexible peptide-protein docking method was used (Kurcinski M et al. (2020) Protein Science, 29: 211-222) for modeling the structure of the complex formed between the peptide identified as SEQ ID NO: 1 and the TTR protein. The crystallographic structure at 1,6 Å resolution of the protein TTR was used (file 3CFM of the Protein Data Bank, PDB), corresponding to the TTR protein (without ligand) and with the wild type sequence. The file contains a dimer of the TTR protein in the asymmetric unit, so the tetramer was obtained by applying the symmetry operations of BIOMOLECULE, described in the pdb file. The two chains symmetric to chains A and B - the original chains of the pdb file - were named C and D, respectively.

The docking protocol used is "blind," meaning that all patches on the surface of the protein constitute potential binding sites. Figure 5A shows the ten superimposed best models proposed by the CABS-dock algorithm. Arrows indicate the location of the peptide identified as SEQ ID NO: 1, bound to the protein in the models obtained. All models indicate that the most likely binding site is located in one of the two tetramer clefts: a) the cleft formed between the monomers of the dimer (inter-monomer cleft: "imc") which is delineated by the extended *beta* sheet of concave exposed face or b) the cleft formed in the space between dimers (inter-dimer cleft: "idc"), delineated by the antiparallel convex beta sheets. The "idc" clefts form the mouth of the channel or binding site of the natural T4 ligand of the TTR protein. This result suggests that these surface patches have stereochemical favorable properties with respect to the rest of the protein surface, such that they constitute potential "attractors" of interactions of the peptide identified as SEQ ID NO: 1. The two "imc" sites are identical to each other (as are the two "idc" sites), as they are related by the C2 symmetry operation. The results indicate that 60% of the models locate the peptide identified as SEQ ID NO: 1 attached to the "idc" site and the remaining 40% on the "imc" site.

Figure 5B shows a model of the peptide identified as SEQ ID NO: 1 attached to the "idc" site. In this model, the most hydrophobic segment of the peptide identified as SEQ ID NO: 1 (residues: Leu15-Ala20) is projected into the mouth of the channel, through interactions with hydrophobic residues of the TTR protein, which contributes favorably to the interaction energy (and the binding affinity), by means of the "hydrophobic effect." As shown in Figure 5C, the deepest region of the "idc" site in contact with the peptide identified as SEQ ID NO: 1 is hydrophobic in nature. The electrostatic complementarity between the peptide identified as SEQ ID NO: 1 and the protein is also reasonable, as shown in Figure 5D. The residue of the peptide identified as SEQ ID NO: 1 that is projected further into the cleft is the Leu18. Figure 5E shows that this leucine residue of the peptide has close atomic contacts with the residues Leu17, Leu103, and Ala108 of the protein. Hydrophobic interactions are observed characteristic of the binding of peptide SEQ ID: 1 to the protein.

On the other hand, Figure 6 shows how the segment Ile11-Ala20 of the peptide, which contains the most hydrophobic residues, is the segment that makes the largest number of contacts with the TTR protein. It reinforces the importance of the hydrophobic effect with respect to energy contribution, fundamental in peptide-protein interactions.

From the functional point of view, the binding of the peptide to the "idc" site contributes to the structural stabilization of the tetramer and in this way can lead to the inhibition and/or modulation of the negative effects of diseases associated with destabilization of the native quaternary structure of the TTR protein. On the other hand, the model is consistent with a central role of the residue Leu18 in the interaction, which results from the substitution of the residue Asp18 present in the native sequence of the T epitope of the protein HSP60 (Barberá A, Lorenzo N, van Kooten P, et al. (2016) Cell Stress and Chaperones.; 21:735-744). The interactions of the residue Leu18 described here could form the structural basis for the specific biological activity of the peptide identified as SEQ ID NO: 1, not observed in the native peptide.

### Example 5. Treatment of patients with ischemic heart disease with pharmaceutical composition comprising the peptide identified as SEQ ID NO: 1.

Patients with low HDL levels and high blood pressure very often develop ischemic heart disease, associated with the formation of atheromatous plaques that characterize the disease called atherosclerosis. These heart diseases often lead patients to a state of severity, and treatment with the peptide identified as SEQ ID NO: 1 may contribute to their recovery.

It is exemplified with the results of six patients. They presented ischemic heart disease, associated with high blood pressure, which produced a state of severity. They were treated in the Intermediate Care Unit (IMCU) of the "Luis Díaz Soto" hospital in Havana. These patients were treated with a pharmaceutical composition comprising the peptide identified as SEQ ID NO: 1, so that a peptide dose of 1 mg every 12 hours was administered intravenously for seven days. In addition, they received the standard therapy needed for these cases.

Figure 7 shows the cholesterol levels in these six patients with ischemic heart disease and high blood pressure treated with the peptide. As can be seen, blood cholesterol levels decreased after seven days of treatment. These patients recovered, and were discharged. These results indicate that treatment with the peptide identified as SEQ ID NO: 1 can stabilize HDL and promote adequate cholesterol turnover, thereby reducing atheromatous plaque, improving atherosclerosis and avoiding associated complications. The treatment with this peptide was very safe, and verified by monitoring blood biochemistry parameters and differential blood count. No side effects were observed in the treated patients, according to clinical and imaging evaluation.

In contrast, five patients with similar characteristics, who did not receive the peptide treatment in the same period of time, had a longer stay in the IMCU, and cholesterol levels in these cases exceeded 5 mmol/L at the end of treatment.

### Example 6. Treatment of patients with type II diabetes with pharmaceutical composition comprising the peptide identified as SEQ ID NO: 1.

Obese and high blood pressure patients very often develop type II diabetes, associated with imbalances of lipid metabolism. These conditions can lead to a serious state, and treatment with the peptide identified as SEQ ID NO: 1 may contribute to their recovery.

It is exemplified with ten patients who had diabetic decompensation and reached a state of severity, that let them to be treated in the ICU of the hospital "Luis Díaz Soto" in Havana. Five of these patients were treated with a pharmaceutical composition comprising the peptide identified as SEQ ID NO: 1, so that a peptide dose of 1 mg every 12 hours was administered intravenously for eight days. In addition, they received the standard therapy needed for these cases.

Figure 8 shows cholesterol levels in patients with type II diabetes who were treated with the peptide, compared to patients who received standard treatment for these cases, but not including the peptide. As can be seen, cholesterol levels remain constant in patients treated with the peptide, compared to untreated patients with the peptide, in whom cholesterol levels exceed normal values at the end of the evaluation period of 8 days. These results indicate that treatment with peptide identified as SEQ ID NO: 1 can stabilize HDL, and promote adequate cholesterol turnover, which contributes to the stability of glycemic metabolism. Treatment with this peptide was very safe and was verified by monitoring blood biochemistry parameters and differential blood count. No side effects were observed in the treated patients, according to clinical and imaging evaluation.

## Claims

1. Peptide identified as SEQ ID NO: 1 for use in the manufacture of a medicine for the treatment of a disease involving an affection of apolipoprotein A-I (Apo-AI) or transthyretin (TTR).

2. The peptide for use according to claim 1 where the disease is **characterized by** an affection in lipid homeostasis or in the stability of TTR.

3. The peptide for use according to claim 2 where the disease is selected from the group composed of atherosclerosis, ischemic heart disease, type II diabetes, secondary dyslipidemia and metabolic syndrome.

4. The peptide for use according to claim 2 where the disease is TTR amyloidosis or Parkinson's disease.

5. A method of treatment of a disease that occurs with an affection in apolipoprotein A-I (Apo-AI) or in transthyretin (TTR) in an individual who needs it, **characterized in that** a therapeutically effective amount of a pharmaceutical composition comprising the peptide identified as SEQ ID NO: 1 is administered.

6. The method of treatment according to claim 5 where the disease is selected from the group composed of atherosclerosis, ischemic heart disease, type II diabetes, secondary dyslipidemia and metabolic syndrome.

7. The method of treatment according to claim 5 where the disease is TTR amyloidosis or Parkinson's disease.

8. The method of treatment according to claim 5 where such pharmaceutical composition is administered by systemic route, preferably by subcutaneous or intravenous route.

9. The method of treatment according to claim 5 where such pharmaceutical composition is administered concurrently with standard therapy in the course of the same treatment.
